# EUROPÄISCHE PATENTANMELDUNG

(11) **EP 1 038 974 A1**
(43) Veröffentlichungstag der Anmeldung: **27.09.2000**
(21) Anmeldenummer: 99106315.7
(22) Anmeldetag: 26.03.1999
(51) Int. Cl.: C12Q 1/68

(54) **Verfahren, Oligonucleotide zum Abbau von in-vitro synthetisierten Nucleinsäuremolekülen**

(71) Anmelder: Mira Diagnostica GmbH, 51377 Leverkusen (DE)
(72) Erfinder: Leiser, Robert-Matthias, 42697 Solingen (DE); Plobner, Lutz, 40699 Erkrath (DE); Temper, Jochen, 51375 Leverkusen (DE); Zavriev, Sergei Kiriakovich, 125422 Moskau (RU); Alexseev, Jakov Igorevich, 121596 Moskau (RU)
(74) Vertreter: Meyers, Hans-Wilhelm, Dr.Dipl.-Chem.

(57) **Zusammenfassung**

Verfahren zum Abbau von in-vitro synthetisierten Nucleinsäuremolekülen mit den Schritten
- Einbau von mindestens einem Derivat von Nucleotidresten in mindestens ein für die Amplifikation notwendiges Starter-Oligonucleotid,
- Durchführung der Amplifikation und Einbau der/des Starteroligonucleotide(s) in die Amplifikationsprodukte und nach Beendigung der Amplifikation
- Abbau der Starter-Oligonucleotid-Sequenzen und deren Komplementärsequenzen,
wobei der Abbau der Starter-Oligonucleotid-Sequenzen und deren Komplementärsequenzen folgende Schritte umfaßt:
- Abspaltung der Base des mindestens einen Derivates mittels einer Nucleinsäureglycosylase, die das mindestens eine Derivat erkennt,
- Schaffung von Strangbrüchen an der (den) Stelle(n), an der (denen) die Base(n) abgespalten worden ist (sind),
- Trennung der/des durch die Abspaltung entstandenen Oligonucleotide(s) von den Komplementärsträngen der Amplifikationsprodukte und
- Entfernung (Hydrolyse) des (der) entstandenen überhängenden 3'-Endes (en) der Amplifikationsprodukte durch eine Nuclease, die 3'-überhängende Enden eines Nucleinsäurestranges abspalten kann.

## Beschreibung

Gegenstand der vorliegenden Erfindung sind Verfahren und Oligonucleotide zum Abbau von in-vitro synthetisierten Nucleinsäuremolekülen.

US-A-5,035,996 und US-A-5,683,896 (Life Technologies) offenbaren Kontaminationen in Reaktionsansätzen zur DNA-Amplifikation dadurch zu kontrollieren, daß in die verwendeten Starter-Oligonucleotide bestimmte Derivate von Nucleotidresten (z. B. Desoxyuridin) eingebaut werden. Vor einer neuen Amplifikationsreaktion können dann eventuell im Reaktionsansatz befindliche Amplifikationsprodukte aus vorangegangenen Amplifikationen dadurch entfernt werden, daß durch Verwendung von geeigneten Enzymen (z. B. Uracil-DNA-Glycosylase, UDG) Strangbrüche in den Amplifikationsprodukten an der Stelle erzeugt werden, an der sich bei den vorangegangenen Amplifikationen die Derivate von Nucleotidresten in den Starter-Oligonucleotiden befanden.

WO 92/01814 (Cetus) offenbart, ein Nucleotidderivat bei der DNA-Synthese in Form des Triphosphats anstelle eines normalen dNTP's (z.B. dUTP anstelle dTTP) randomisiert einzubauen. Vor einer neuen Amplifikation wird verfahren wie in den oben genannten Life Technologies Veröffentlichungen.

Das Verfahren nach Life Technologies hat gegenüber WO 92/01814 den Vorteil, daß durch den gezielten Einbau der Nucleotidderivate in die Starter-Oligonucleotide die UDG nicht das gesamte Amplifikat zerstört, sondern nur die Abschnitte abgetrennt werden, die nötig sind, damit in einer neuen Amplifikationsreaktion die DNA durch neue Starter-Oligonucleotide nicht erkannt wird. Dies hat insbesondere den Vorteil, daß man entstandene Amplifikate so verändern kann, daß sie nicht mehr als Vorlage (Template) für weitere Amplifikationen dienen können, jedoch immer noch detektierbar bleiben. Ein zweiter Vorteil besteht darin, daß die häufig auftretende partielle Syntheseinhibition und der dadurch bedingte Sensitivitätsverlust als Folge des zumindest teilweisen Ersatzes des dTTP durch dUTP umgangen wird.

Untersuchungen haben ergeben, daß das Verfahren nach Life Technologies nicht funktionell ist. Dafür sind in Abhängigkeit von den konkreten Verfahrensbedingungen unterschiedliche Gründe verantwortlich.

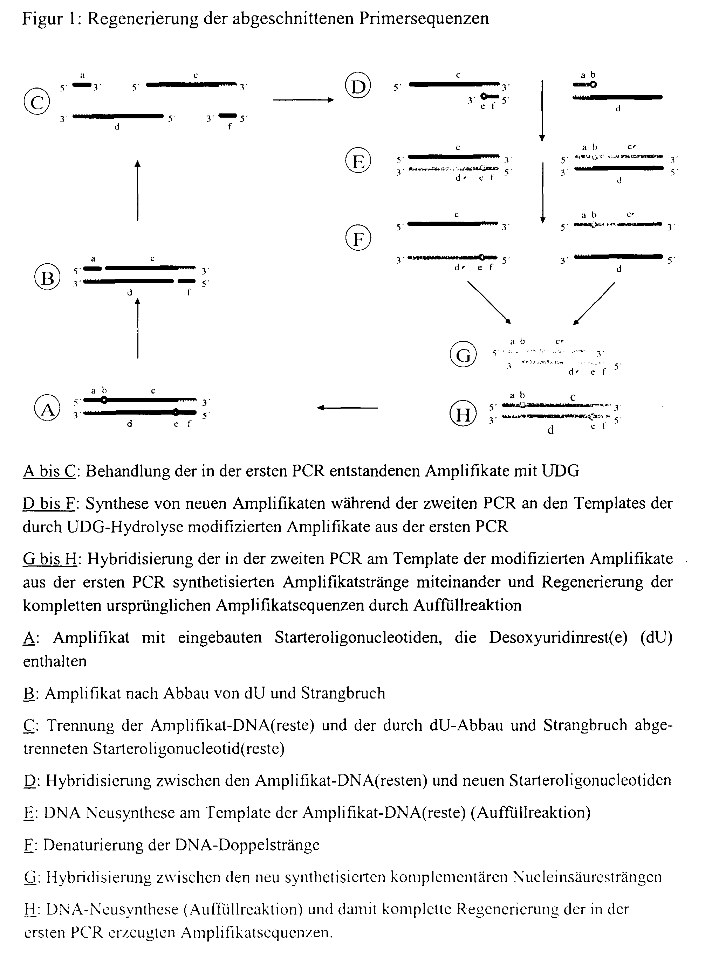

Ein wichtiger Grund für das Fehlschlagen des Life Technologies Verfahrens besteht möglicherweise in folgendem: Die Nucleotidderivate werden als Bestandteil der Starter-Oligonucleotide jeweils in der Nähe der 5'-Enden der Amplifikationsprodukte eingebaut. Durch Strangbruch und Abtrennung dieses Oligonucleotids werden aber eventuelle Kontaminationen in neuen Amplifikations-Reaktionsansätzen nicht wirksam zerstört, weil zwar jeweils die 5'-Enden des Amplifikationsproduktes gekürzt werden, diese aber durch mehrere Schritte von Denaturierung, Renaturierung und Strangverlängerung, wie sie bei erneuter PCR ablaufen, wieder regeneriert werden können. Dies veranschaulicht Figur 1.

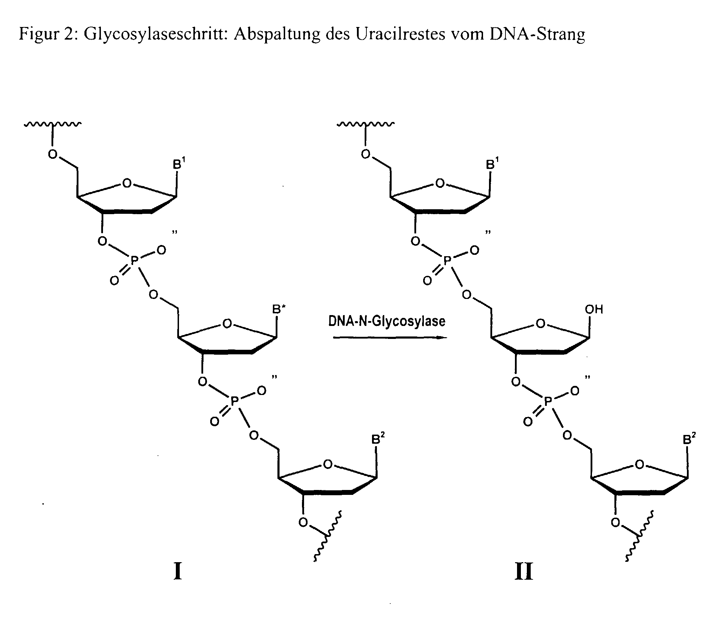

Weitere Gründe des Fehlschlagens dieses Verfahrens können unter bestimmten Bedingungen darin bestehen, daß UDG zwar die Stickstoffbase an der Stelle des Nucleotidderivat-Einbaus angreift, aber selbst keinen Strangbruch verursacht (David und Williams, 1998; Krokan et al., 1997). Dies veranschaulicht Figur 2.

Das der Erfindung zugrundeliegende Problem bestand darin, die oben genannten Nachteile zu vermeiden.

Die vorliegende Erfindung führt zu einem brauchbaren Verfahren zur Kontaminationskontrolle von Amplifikationsreaktionen.

Das erfindungsgemäße Verfahren zum Abbau von in-vitro synthetisierten Nucleinsäuremolekülen weist die folgenden Schritte auf:
- Einbau von mindestens einem Derivat von Nucleotidresten in mindestens ein für die Amplifikation notwendiges Starter-Oligonucleotid,
- Durchführung der Amplifikation und Einbau der/des Starter-Oligonucleotide(s) in die Amplifikationsprodukte und nach Beendigung der Amplifikation
- Abbau der Starter-Oligonucleotid-Sequenzen und deren Komplementärsequenzen,
wobei der Abbau der Starter-Oligonucleotid-Sequenzen und deren Komplementärsequenzen folgende Schritte umfaßt:
- Abspaltung der Base des mindestens einen Derivates mittels einer Nucleinsäureglycosylase, die das mindestens eine Derivat erkennt,
- Schaffung von Strangbrüchen an der (den) Stelle(n), an der (denen) die Base(n) abgespalten worden ist (sind),
- Trennung der/des durch die Abspaltung entstandenen Oligonucleotide(s) von den Komplementärsträngen der Amplifikationsprodukte und
- Entfernung (Hydrolyse) des (der) entstandenen überhängenden 3'-Endes (en) der Amplifikationsprodukte durch eine Nuclease, die 3'-überhängende Enden eines Nucleinsäurestranges abspalten kann.

In dem erfindungsgemäßen Verfahren werden vorzugsweise als Amplifikationsprodukte in-vitro synthetisierte Nucleinsäuremoleküle verwendet. Vorzugsweise handelt es sich bei den Amplifikationsprodukten um in-vitro ligierte Nucleinsäuremoleküle.

Insbesondere wird erfindungsgemäß Desoxyuridin als Derivat von Nucleotidresten in die Starter-Oligonucleotide eingebaut.

Die Starter-Oligonucleotide weisen vorzugsweise mindestens eine Struktureinheit auf, die mindestens eine Erkennungsstelle für Nucleasen, Glycosylasen, und/oder Methylasen und mindestens eine transformierbare Gruppe aufweist, die nach einer Transformation die Erkennungsstelle freilegt.

Als Transformation wird hier eine Abspaltungsreaktion verstanden, die chemisch, insbesondere photolytisch, oder biochemisch, insbesondere enzymatisch ist. Die Struktureinheit in den erfindungsgemäßen Verfahren zu verwendenden Oligonucleotiden wird beispielsweise durch einen atypischen Nucleosidrest, der aus einer atypischen Base, die C-N-glycosidisch mit einem Zuckerbaustein der Gruppe der Pentosen und Hexosen bzw. Desoxypentosen und Desoxyhexosen verknüpft ist, gebildet. Die Erkennungsstelle in den Oligonucleotiden zur Verwendung im erfindungsgemäßen Verfahren ist eine modifizierte Base in der Nucleinsäurekette, vorzugsweise der folgenden Struktur: Uracil, 5-Uracilcarbonsäure, Hypoxanthin, 5-Formyluracil, 5-Hydroxymethylcytosin oder 5-Hydroxymethyluracil.

Als "atypische Base" wird erfindungsgemäß folgende Verbindung aufgefaßt: 5-(2-(1,3-Dioxanyl))-uracil, 5-(2-Nitrobenzoxy)methylcytosin, 5-(4-Methoxy-2-nitrobenzoxy)methylcytosin, 5-(4,5-Dimethoxy-2-nitrobenzoxy)methylcytosin [= 5-(6-Nitroveratryloxy)methylcytosin], 5-(2-Nitrobenzoxy)methyluracil, 5-(4-Methoxy-2-nitrobenzoxy)methyluracil, 5-(4,5-Dimethoxy-2-nitrobenzoxy)methyluracil [= 5-(6-Nitroveratryloxy)methyluracil], 5-(2-(4-(2-Nitrophenyl)-1,3-dioxola-nyl))-methyluracil, 6-(2-Nitrobenzoxy)purin, 6-(4-Methoxy-2-nitrobenzoxy)purin, 6-(4,5-Dimethoxy-2-nitrobenzoxy)purin [= 5-(6-Nitroveratryloxy)purin].

Vorzugsweise handelt es sich bei der Nucleinsäureglycosylase um Uracil-DNA-Glycosylase.

Die Zugabe der Nucleinsäureglycosylase zum Abbau der Starter-Oligonucleotid-Sequenzen und deren Komplementärsequenzen kann beispielsweise zum Reaktionsgemisch der ursprünglichen Amplifikation oder auch zum Reaktionsgemisch vor Beginn der neuen Amplifikation erfolgen.

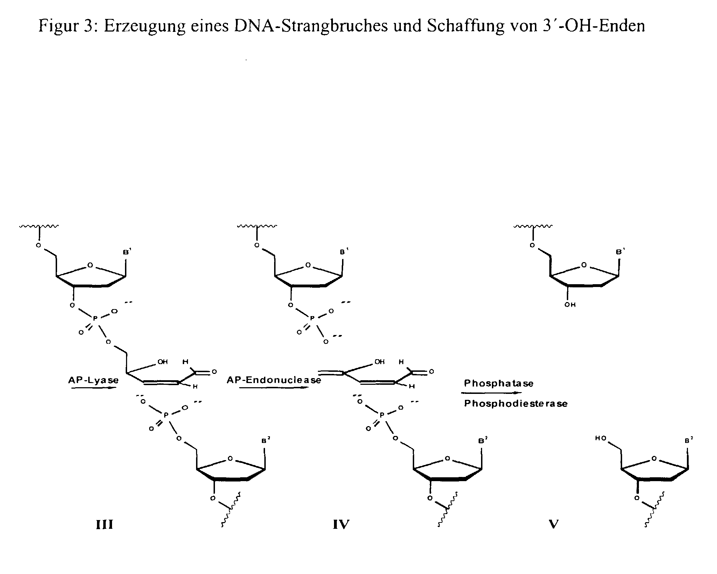

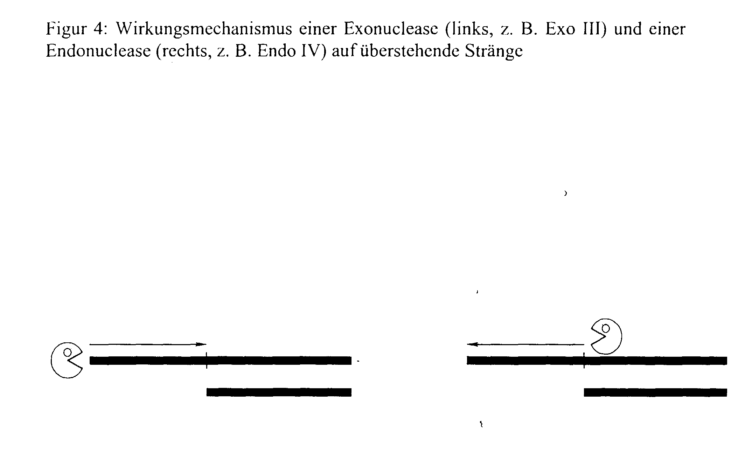

Die Erzeugung von Strangbrüchen an den durch die Glykosylase geschaffenen Halbacetalen bzw. Hydroxyaldehyden und Ablösung der dabei abgetrennten, aber noch in Basenpaarung mit dem Komplementärstrang befindlichen Oligonucleotiden kann durch erhöhte Temperatur, insbesondere über 50°C unter alkalischen Bedingungen z.B. pH-Wert über 8,2 ablaufen. In anderen Fällen ist es vorteilhaft, die Strangbrüche durch entsprechende Lyaseaktivität zu schaffen und die wirksame Freisetzung der abgetrennten Oligonucleotide dadurch zu gewährleisten, daß an deren 3'-Ende freie 3'-OH-Enden durch Einsatz einer Phosphatase- oder Phosphodiesterase-Aktivität kreiert werden (Figur 3). Dadurch kann die zuvor genannte 3'-5'-Exonuclase sukzessive die abgetrennten, aber noch in Basenpaarung mit dem Komplementärstrang befindlichen Oligonucleotide abbauen und die dadurch entstehenden 3'-überhängenen DNA-Stränge angreifen. Dies veranschaulicht Figur 4.

Die Strangbrüche können erfindungsgemäß auch durch Verwendung einer Lyase geschaffen werden.

Erfindungsgemäß erfolgt die Ablösung des abgespaltenen Oligonucleotids beispielsweise durch Erwärmen auf Temperaturen oberhalb des Schmelzpunktes der entstandenen Oligonucleotide. Zur Ablösung des abgespaltenen Oligonucleotids können erfindungsgemäß auch dessen 3'-Enden in freie 3'-OH-Enden umgeformt werden, wobei die Ablösung vom komplementären Strang dann durch enzymatischen Abbau, insbesondere durch Phosphatasen oder Phosphodiesterasen oder 3'-5'-Exonucleasen erfolgt.

Die notwendige 3'-5'-Exonuclease-Aktivität kann verschiedenen Ursprungs sein. Es kann z.B. eine DNA-Polymerase zur Amplifikation verwendet werden, die selbst eine solche Aktivität aufweist (z.B. Pfu-DNA-Polymerase), oder es kann eine solche DNA-Polymerase mit 3'-5'-Exonuclease-Aktivität im Gemisch z.B. mit der in PCR-Reaktionen häufig verwandten Taq- bzw. Tth-DNA-Polymerase eingesetzt werden. Schließlich kann eine 3'-5'-Exonuclease ohne eigene DNA-Polymerase-Aktivität eingesetzt werden. Bei letzteren handelt es sich insbesondere um natürliche Nucleasen (z.B. Exonuclease III, Exonuclease VII oder Endonuclease IV), die auch rekombinant gewonnen werden können, oder aber um Mutanten von DNA-Polymerasen mit 3'-5'-Exonuclease-Aktivität, bei denen die Polymeraseaktivität verändert wurde.

Die Lyase- und eventuell auch Phosphatase- oder Phosphodiesterase-Aktivität kann dadurch gewährleistet werden, daß bestimmte Glycosylasen zum Einsatz gelangen, die über solche zusätzlichen Enzymaktivitäten verfügen (z.B. NTG1, NTH, FPG, OGG1, OGG2, T4-Endonuclease V, Endonuclease III) oder aber daß derartige Enzyme zum Reaktionsansatz zugesetzt werden (z.B. Exonuclease III, Endonuclease IV, HAPI, Apn1).

Die erfindungsgemäßen Oligonucleotide, die im erfindungsgemäßen Verfahren eingesetzt werden können, enthalten mindestens ein Derivat von Nucleotidresten, deren Stickstoffbase durch eine Nucleinsäureglycosylase abspaltbar ist. Vorzugsweise ist mindestens eine Struktureinheit vorgesehen, die mindestens eine Erkennungsstelle für Nucleasen, Glycosylasen, und/oder Methylasen und mindestens eine transformierbare Gruppe aufweist, die nach einer Transformation die Erkennungsstelle freilegt.

Die transformierbare Gruppe ist vorzugsweise so gestaltet, daß sie mit einer chemischen, insbesondere photolytischen, oder biochemischen, insbesondere enzymatischen Reaktion eine Erkennungsstelle freilegt. Die Struktureinheit im erfindungsgemäßen Oligonucleotid ist ein atypischer Nucleosidrest, der aus einer atypischen Base, die C-N-glycosidisch mit einem Zuckerbaustein der Gruppe der Pentosen und Hexosen bzw. Desoxypentosen und Desoxyhexosen verknüpft ist. Dabei ist die Erkennungsstelle insbesondere eine modifizierte Base in der Nucleinsäurekette, insbesondere Uracil, 5-Uracilcarbonsäure, Hypoxanthin, 5-Formyluracil, 5-Hydroxymethylcytosin, 5-Hydroxymethyluracil.

Als "atypische Base" werden in den erfindungsgemäßen Oligonucleotiden die folgenden Verbindungen eingesetzt: 5-(2-(1,3-Dioxanyl))-uracil, 5-(2-Nitrobenzoxy)methylcytosin, 5-(4-Methoxy-2-nitrobenzoxy)methylcytosin, 5-(4,5-Dimethoxy-2-nitrobenzoxy)methylcytosin [= 5-(6-Nitroveratryloxy)methylcytosin], 5-(2-Nitrobenzoxy)methyluracil, 5-(4-Methoxy-2-nitrobenzoxy)methyluracil, 5-(4,5-Dimethoxy-2-nitrobenzoxy)methyluracil [= 5-(6-Nitroveratryloxy)methyluracil], 5-(2-(4-(2-Nitrophenyl)-1,3-dioxolanyl))methyluracil, 6-(2-Nitrobenzoxy)purin, 6-(4-Methoxy-2-nitrobenzoxy)purin, 6-(4,5-Dimethoxy-2-nitrobenzoxy)purin [= 5-(6-Nitroveratryloxy)purin].

Die Erfindung wird anhand des folgenden Ausführungsbeispiels näher erläutert:

### Beispiel

Zur Durchführung der PCR werden folgende Reaktionskomponenten miteinander vermischt:

| | |
|---|---|
| 10x PCR-Puffer | |
| (0,1% Tween, 660mM Tris-HCl pH 8,8, 166mM (NH₄)₂SO₄ | 5,0µl |
| MgCl₂ (25mM) | 5,0µl |
| dNTP's (je 2mM) | 5,0µl |
| DNA-Polymerase (5U/µl) | 0,2µl |
| DNA-Template (0,2ng/µl) | 5,0µl |
| Primer A (30pmol) | 1,0µl |
| Primer B (30pmol) | 1,0µl |
| H₂O | 27,8µl |

Die Amplifikation der Ziel-DNA (Target-DNA) erfolgt in einem Thermocycler (GeneAmp PCR System 9700 von Perkin Elmer) mit vorzugsweise beheiztem Deckel. Hierbei wird die DNA zuerst für 5min bei 94°C denaturiert und anschließend für 30 Zyklen unter folgenden Bedingungen amplifiziert:

| | | |
|---|---|---|
| 30sec | 94°C | Zyklus-Denaturierung |
| 15sec | 56°C | Primer-Hybridisierung ("Annealing") |
| 20sec | 72°C | Elongation ("Extension") |

Zum Abschluß des PCR-Programmes erfolgt eine nochmalige Elongation bei 72°C für 1min.

### Ausführungsbeispiel 1

| Abkürzungen: | |
|---|---|
| UDG | Uracil-DNA-Glycosylase |
| dU | 2'-Desoxyuridin |
| dUCP | 5'-Dimethoxytrityl-dU-3'-[(2-cyanethyl)-(N,N-diisopropyl)]-phosphoramidit |

Die Oligonucleotide werden im Syntheseautomaten nach den dem Gerät entsprechenden Vorschriften synthetisiert. Der Einbau des dUCP erfolgt dabei an jeweils drei Thymin-Positionen der Primer.

Nach einer sich anschließenden PCR (entsprechend dem vorstehenden Beispiel) wird ein Enzymgemisch aus 1 µl UDG (1 Unit/100) und 0,5 µl Pfu-DNA-Polymerase (5 Unit/µl) zugegeben und erhitzt für 10 min bei 37°C.

### Beispiel 2

Synthese der Oligonucleotide und Durchführung der PCR erfolgt wie oben beschrieben. Nach der PCR-Amplifikation wird ein Enzymgemisch aus 1 µl UDG (1 Unit/100), 2 µl Exonuclease III (1 unit/100 µl) sowie 2 µl Exonuclease VII (1 unit/100 µl). Man inkubiert für 30 min bei 31°C.

## Patentansprüche

1. Verfahren zum Abbau von in-vitro synthetisierten Nucleinsäuremolekülen mit den Schritten
• Einbau von mindestens einem Derivat von Nucleotidresten in mindestens ein für die Amplifikation notwendiges Starter-Oligonucleotid,
• Durchführung der Amplifikation und Einbau der/des Starteroligonucleotide(s) in die Amplifikationsprodukte und nach Beendigung der Amplifikation
• Abbau der Starter-Oligonucleotid-Sequenzen und deren Komplementärsequenzen,
wobei der Abbau der Starter-Oligonucleotid-Sequenzen und deren Komplementärsequenzen folgende Schritte umfaßt:
• Abspaltung der Base des mindestens einen Derivates mittels einer Nucleinsäureglycosylase, die das mindestens eine Derivat erkennt,
• Schaffung von Strangbrüchen an der (den) Stelle(n), an der (denen) die Base(n) abgespalten worden ist (sind),
• Trennung der/des durch die Abspaltung entstandenen Oligonucleotide(s) von den Komplementärsträngen der Amplifikationsprodukte und
• Entfernung (Hydrolyse) des (der) entstandenen überhängenden 3'-Endes (en) der Amplifikationsprodukte durch eine Nuclease, die 3'-überhängende Enden eines Nucleinsäurestranges abspalten kann.

2. Verfahren nach Anspruch 1, gekennzeichnet dadurch, daß es sich bei den Amplifikationsprodukten um in-vitro synthetisierte Nucleinsäuremoleküle handelt.

3. Verfahren nach Anspruch 1, gekennzeichnet dadurch, daß es sich bei den Amplifikationsprodukten um in-vitro ligierte Nucleinsäuremoleküle handelt.

4. Verfahren nach Anspruch 1 bis 3, gekennzeichnet dadurch, daß es sich bei den in die Starter-Oligonucleotide eingebauten Derivaten von Nucleotidresten um Desoxyuridin handelt.

5. Verfahren nach Anspruch 1 bis 3, gekennzeichnet dadurch, daß in den Starter-Oligonucleotiden mindestens eine Struktureinheit vorgesehen ist, die mindestens eine Erkennungsstelle für Nucleasen, Glycosylasen, und/oder Methylasen und mindestens eine transformierbare Gruppe aufweist, die nach einer Transformation die Erkennungsstelle freilegt.

6. Verfahren nach Anspruch 1 bis 4, gekennzeichnet dadurch, daß es sich bei der Nucleinsäureglycosylase um Uracil-DNA-Glycosylase handelt.

7. Verfahren nach Anspruch 1 bis 6, gekennzeichnet dadurch, daß der Abbau der Starter-Oligonucleotid-Sequenzen und deren Komplementärsequenzen durch Zugabe der Nucleinsäureglycosylase zum Reaktionsgemisch der ursprünglichen Amplifikation erfolgt.

8. Verfahren nach Anspruch 1 bis 6, gekennzeichnet dadurch, daß der Abbau der Starter-Oligonucleotid-Sequenzen und deren Komplementärsequenzen durch Zugabe der Nucleinsäureglycosylase zum Reaktionsgemisch vor Beginn der neuen Amplifikation erfolgt.

9. Verfahren nach Anspruch 1 bis 8, gekennzeichnet dadurch, daß die Schaffung von Strangbrüchen und Ablösung des abgespalteten Oligonucleotids durch Erwärmung auf Temperaturen über 50°C im alkalischen Milieu (pH-Wert über 8,2) geschieht.

10. Verfahren nach Anspruch 1 bis 8, gekennzeichnet dadurch, daß die Schaffung von Strangbrüchen mittels einer Lyase erfolgt.

11. Verfahren nach Anspruch 1 bis 8 und 10, gekennzeichnet dadurch, daß die Ablösung des abgespalteten Oligonucleotids durch Erwärmen auf Temperaturen oberhalb des Schmelzpunktes der entstandenen Oligonucleotide geschieht.

12. Verfahren nach Anspruch 1 bis 8 und 10, gekennzeichnet dadurch, daß zur Ablösung des abgespalteten Oligonucleotids dessen 3'-Enden in freie 3'-OH-Enden umgeformt werden und ihre Ablösung vom Komplementärstrang durch enzymatischen Abbau, insbesondere durch Phosphatase oder Phosphodiesterase oder durch 3'-5'-Exonuclease erfolgt.

13. Verfahren nach Anspruch 12, gekennzeichnet dadurch, daß es sich bei der 3'-5'-Exonuclease um DNA-Polymerase I aus E. coli, T4-DNA-Polymerase, um thermostabile DNA-Polymerase mit entsprechender 3'-5'-Exonuclease-Aktivität handelt wie Taq-, Tth- oder Pfu-DNA-Polymerase.

14. Verfahren nach Anspruch 13, gekennzeichnet dadurch, daß die DNA-Polymeraseaktivität der verwendeten Enzyme mit 3'-5'-Exonucleaseaktivität verändert wurde.

15. Oligonucleotide zum Einsatz in einem Verfahren nach Anspruch 1 bis 14 gekennzeichnet dadurch, daß sie mindestens ein Derivat von Nucleotidresten enthalten, deren Stickstoffbase durch eine Nucleinsäureglycosylase abgespalten werden kann.

16. Oligonucleotide nach Anspruch 15 gekennzeichnet dadurch, dass in ihnen mindestens eine Struktureinheit vorgesehen ist, die mindestens eine Erkennungsstelle für Nucleasen, Glycosylasen, und/oder Methylasen und mindestens eine transformierbare Gruppe aufweist, die nach einer Transformation die Erkennungsstelle freilegt, verwendet wird.
